# EUROPÄISCHE PATENTSCHRIFT

(11) **EP 0 915 893 B1**
(45) Veröffentlichungstag und Bekanntmachung des Hinweises auf die Patenterteilung: **14.11.2001**
(21) Anmeldenummer: 97940008.2
(22) Anmeldetag: 21.07.1997
(51) Int. Cl.: C07F 9/50, C08G 65/26, C07C 45/50

(54) **ALKYLENGLYKOLGRUPPEN ENTHALTENDE TERTIÄRE PHOSPHANE**
TERTIARY PHOSPHANES CONTAINING ALKYLENE GLYCOL GROUPS
PHOSPHANES TERTIAIRES CONTENANT DES GROUPES ALKYLENEGLYCOLS

(30) Priorität: 29.07.1996 DE 19630534; 30.04.1997 DE 19718196
(43) Veröffentlichungstag der Anmeldung: 19.05.1999
(73) Patentinhaber: Celanese Chemicals Europe GmbH, 60439 Frankfurt am Main (DE)
(72) Erfinder: HABER, Steffen, D-61462 Königstein (DE); KLEINER, Hans-Jerg, D-61476 Kronberg (DE); BOGDANOVIC, Sandra, D-60322 Frankfurt (DE); BAHRMANN, Helmut, D-46499 Hamminkeln (DE); FROHNING, Carl-Dieter, D-46485 Wesel (DE)
(86) Internationale Anmeldenummer: EP9703927
(87) Internationale Veröffentlichungsnummer: WO9804568

(56) Entgegenhaltungen:
- TERFORT A ET AL: "Enantioselective catalysis. 99. Phosphine ligands with two binding sites of differing hardness for enantioselective Grignard cross coupling" J. CHEM. SOC., PERKIN TRANS. 1 (JCPRB4,0300922X);96; (12); PP.1467-1479, UNIV. REGENSBURG;INST. ANORGANISCHE CHEM.; REGENSBURG; 93040; GERMANY (DE), XP002043595
- CHEMICAL ABSTRACTS, vol. 125, no. 3, 15.Juli 1996 Columbus, Ohio, US; abstract no. 033125, YAN Y Y ET AL: "Aqueous-phase rhodium hydroformylation of dodecene-1 with surface-active water-soluble phosphine" XP002043597 & CHIN. CHEM. LETT. (CCLEE7);96; VOL.7 (4); PP.377-80, DALIAN UNIV. OF TECHNOLOGY;COLL. OF CHEMICAL ENGINEERING; DALIAN; 116012; PEOP. REP. CHINA (CN),
- TASHIRO M ET AL: "Polyethers. 1. Preparation of.omega.,.omega.'-bis(triphenylphosp hine) polyethers" J. ORG. CHEM. (JOCEAH,00223263);80; VOL.45 (6); PP.1156-8, KYUSHU UNIV. 86;RES. INST. IND. SCI.; FUKUOKA; 812; JAPAN, XP002043596
- CHEMICAL ABSTRACTS, vol. 105, no. 7, 18.August 1986 Columbus, Ohio, US; abstract no. 060677, TIMMER K ET AL: "The synthesis of some polyether bridged diphosphines and their reaction with Rh(COD)acac" XP002043598 & INORG. CHIM. ACTA (ICHAA3,00201693);85; VOL.100 (2); PP.235-40, INST. APPL. CHEM.;UTRECHT; 3502 JA; NETH. (NL),

## Beschreibung

Die vorliegende Erfindung liegt auf dem Gebiet der organischen Phosphorchemie.

Die Erfindung betrifft neue Alkylenglykolgruppen tragende tertiäre Phosphane und deren Herstellung durch nukleophile Substitution.

Komplexverbindungen, die als Zentralatom ein Metall der 8. Nebengruppe des Periodensystems der Elemente und als Liganden P(III)-Verbindungen, z.B. Phosphane oder Phosphite, und gegebenenfalls noch weitere zur Komplexbildung befähigte Gruppen enthalten, finden in den letzten Jahren zunehmend Anwendung als Katalysatoren für organisch-chemische Synthesen. So erfolgt die technisch in großem Umfang ausgeführte Reaktion von Olefinen mit Synthesegas zu Aldehyden (Hydroformylierung) in Gegenwart von Katalysatorsystemen, die aus Kobalt und insbesondere Rhodium und Triphenylphosphan bestehen. Auch zur Umsetzung von Methanol mit Synthesegas zu höheren Alkoholen (Homologisierung) haben sich Katalysatoren auf Basis von Phosphan enthaltenden Komplexverbindungen bewährt. Zumeist liegen in den genannten Fällen die Liganden im Überschuß vor, so daß das Katalysatorsystem aus Komplexverbindung und freiem Ligand besteht. Entsprechend der Löslichkeit der Katalysatoren in organischen Medien erfolgen die Reaktionen in homogener Phase.

Statt in homogener Phase kann man die Umsetzung auch in heterogenen, mehrphasigen Reaktionssystemen durchführen. Ein Vorteil dieser Verfahrensvariante ist die einfache und schonende Trennung des in Wasser gelösten Katalysators von dem in Wasser nicht löslichen Reaktionsprodukt. Nach diesem Prinzip arbeitet z.B. das in der DE-C2-27 00 904 beschriebene Verfahren zur Herstellung von Nitrilen durch Anlagerung von Cyanwasserstoff an ungesättigte organische Verbindungen, die mindestens eine ethylenische Doppelbindung enthalten. Für die Herstellung von Aldehyden durch Reaktion von Olefinen mit Kohlenmonoxid und Wasserstoff setzt man nach dem in der DE-C2-26 27 354 beschriebenen Prozeß Rhodium in metallischer Form oder in Form seiner Verbindungen zusammen mit einem wasserlöslichen Phosphan, z.B. dem Alkalisalz des Tri-(m-sulfonatophenyl)-phosphans ("TPPTS") als Katalysator ein. Weitere Eeispiele für Umsetzungen mit heterogener Katalysatorphase finden sich in Angew. Chem. 1993, 105, 1588 ff.

J. Chem. Soc., Perkin Trans. 1 1996; 1467, lehrt chirale Phosphanliganden, die für die asymmetrische Grignard Kreuzkupplung eingesetzt werden. Chem. Abstracts, vol. 125, no. 33125x, beschreibt Phosphanverbindungen, die drei Polyetherreste pro Phosphoratom enthalten. J. Organ. Chem. 1980, 1156 und Chem. Abstracts, vol. 105, no. 60677x, beziehen sich auf Biphosphanverbindungen, die durch eine Polyetherkette überbrückt werden.

Zweiphasige Verfahren haben sich auch in technischem Maßstab gut bewährt. Arbeiten aus jüngerer Zeit dienen einer weiteren Vervollkommnung der Prozesse. So versucht man, die Aktivität der Katalysatoren durch Modifizieren der Komplexliganden zu erhöhen und ihre Wirksamkeit zu verlängern, um den spezifischen Katalysatorbedarf - sowohl Metall als auch Ligand - und damit die Produktkosten weiter zu senken. Wirtschaftliche Gründe sind auch dafür maßgebend, auf eine deutliche Verminderung des Phosphan/Metall-Verhältnisses hinzuarbeiten. Schließlich bemüht man sich, die im Rahmen bekannter Verfahren produktspezifischen Probleme zu lösen. Darüber hinaus sucht man neue Anwendungsgebiete für diese vielseitigen Katalysatorsysteme.

Der vorliegenden Erfindung lag die Aufgabe zugrunde, neue Phosphane zur Verfügung zu stellen, die insbesondere für den Einsatz in Katalysatorsystemen für organische Synthesen geeignet sind.

Gegenstand der vorliegenden Erfindung sind tertiäre Phosphane der Formel (I) 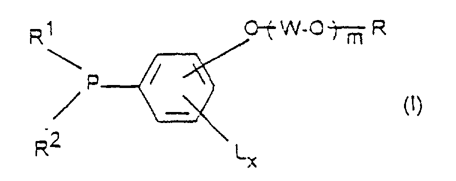 worin
- m: eine Zahl von 2 bis 300, bevorzugt 2 bis 100;
- x: eine Zahl von 0 bis 4, bevorzugt 0 oder 1;
- W: eine Gruppe der Formeln -CH₂-CH₂-, -CH(CH₃)CH₂- oder -CH₂CH(CH₃)-;
- R: Wasserstoff, ein geradkettiger oder verzweigter C₁-C₅-Alkylrest;
oder eine Gruppe der Formeln 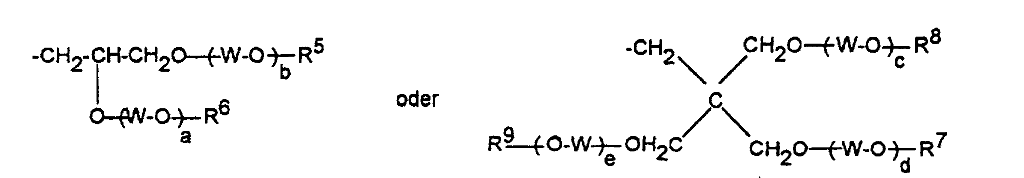 wobei
- a, b, c, d und e: unabhängig voneinander eine Zahl von 0 bis 1000, wobei mindestens eine der Zahlen a, b, c, d und e größer als 0 ist;
- R⁵, R⁶, R⁷, R⁸ und R⁹: gleich oder verschieden sind und Wasserstoff, C₁-C₅-Alkyl oder eine Gruppe der Formel 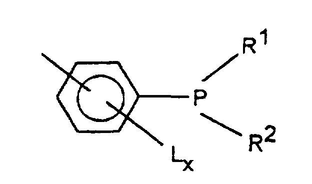 ist,
- R¹ und R²: gleich oder verschieden sind und einen geradkettigen, verzweigten oder cyclischen C₁-C₃₀-Alkylrest oder C₆-C₁₀-Arylrest, der unsubstituiert oder durch ein bis fünf C₁-C₃-Alkylreste substituiert ist, oder R¹ und R² zusammen mit dem dreiwertigen P-Atom ein Dibenzophospholyl der Formel 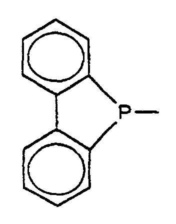 oder ein 3,4-Dimethylphospholyl der Formel 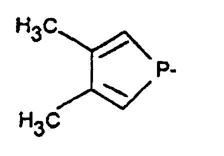 bilden, und
- L: C₁-C₅-Alkyl, C₁-C₅-Alkoxy, NO₂, NR³R⁴, wobei R³ und R⁴ unabhängig voneinander Wasserstoff oder C₁-C₄-Alkyl bedeuten, Cl oder OH bedeuten.

Die Alkylenglykolgruppen am Phenylring können sich in ortho-, meta- oder para- Position zum Phosphoratom befinden. Die der Gruppe -(W-O-)ₘ zugrundeliegende Oxalkylenkette kann ausschließlich aus Ethylenoxid- oder ausschließlich aus Propylenoxid-Einheiten oder aus einer Kombination dieser Einheiten in beliebiger Reihenfolge bestehen.

Von besonderem Interesse sind Verbindungen der Formel (I), worin R¹ und R² gleich sind und jeweils einen geradkettigen oder verzweigten C₁-C₆-Alkylrest, einen Cyclohexylrest oder einen Phenylrest bedeuten.

Von besonderem Interesse sind ferner Verbindungen der Formel (I), worin R Wasserstoff, Methyl, Ethyl, n-Propyl, n-Butyl oder eine Gruppe der Formel 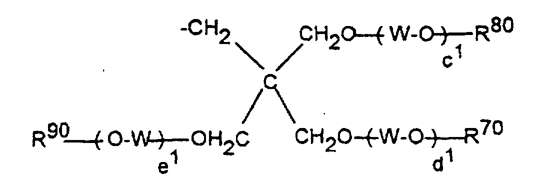 worin c¹, d¹ und e¹ unabhängig voneinander eine Zahl von 1 bis 500, insbesondere 2 bis 300, und
R⁷⁰, R⁸⁰ und R⁹⁰ gleich oder verschieden sind und Wasserstoff, Methyl, Ethyl, n-Propyl oder n-Butyl bedeuten.

Von besonderem Interesse sind ferner Verbindungen der Formel (I), worin L Methoxy, Ethoxy, Methyl, Ethyl oder OH ist oder worin x die Zahl 0 bedeutet.

Beispiele für Verbindungen der Formel (I) sind Methyl-(triphenylphosphin-4-yl)-tri-ethylenglykol-ether, Methyl-(triphenylphosphin-3-yl)-tri-ethylenglykol-ether, Methyl-(triphenylphosphin-2-yl)-tri-ethylenglykol-ether, sowie Verbindungen mit längeren Oxalkylketten, wobei die Ethoxy- und Propoxyeinheiten in beliebiger Reihenfolge stehen und in der Regel ein Produktgemisch bilden: 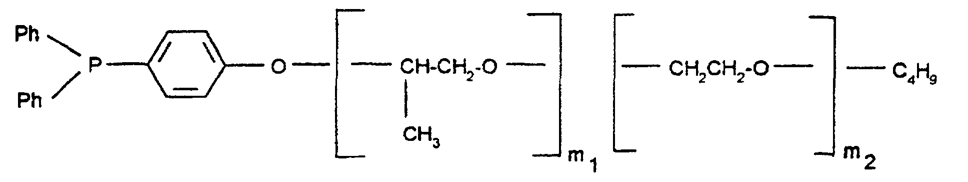 worin m₁ und m₂ jeweils etwa die Zahl 16 und Ph Phenyl bedeuten; 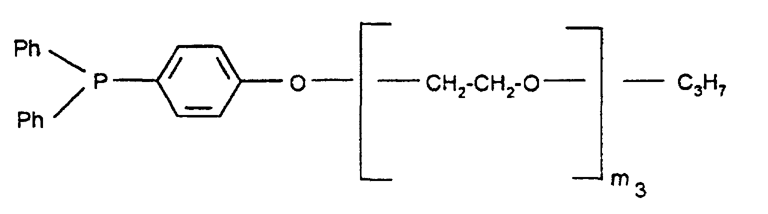 worin m₃ eine Zahl von etwa 22 bedeutet; 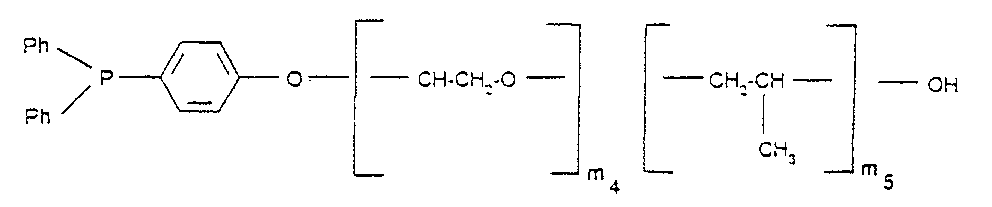 worin m₄ etwa 84 und m₅ etwa 21 bedeutet, 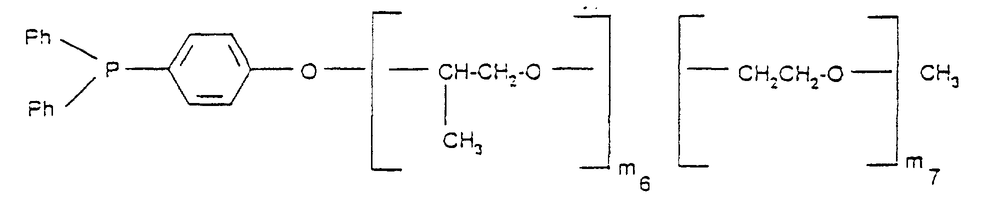 worin m₆ etwa 22 und m₇ 5,5 bedeutet, 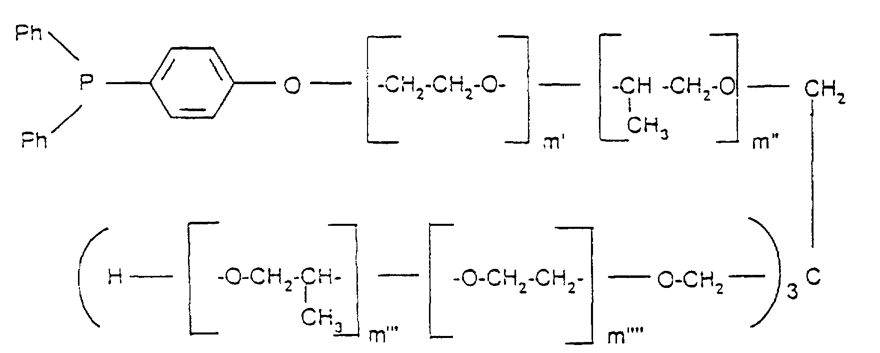 worin m' etwa 10, m" etwa 2 bis 3, m''' etwa 7 bis 8 und m"" etwa 30 bedeutet.

Die Verteilung der Oxalkylengruppen im Produkt, insbesondere das Verhältnis und die Anordnung (Reihenfolge) von Ox-ethylen- zu Ox-2-propylen-Gruppen ist durch die eingesetzten Edukte gegeben. Diese werden durch Anlagerung von Ethylenoxid und Propylenoxid an Wasser beziehungsweise Alkohole hergestellt. Die Anlagerung und Anordnung erfolgt unter statistischen Gesichtspunkten.
Die eingesetzten Edukte sind Verkaufsprodukte der Hoechst AG.

Gegenstand der Erfindung ist auch ein Verfahren zur Herstellung einer Verbindung der Formel (I), dadurch gekennzeichnet, daß man ein Hydroxyphenylphosphan der Formel (II) 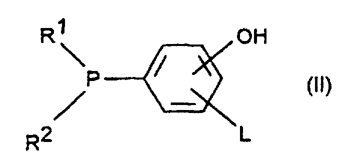 mit einer Base zum entsprechenden Phenolat deprotoniert und mit einer Verbindung der Formel (III)

X-(-W-O-)ₘ-R (III)

worin W, R und m wie vorstehend definiert sind und X eine nucleophil substituierbare Abgangsgruppe bedeutet, zur Verbindung der Formel (I) umsetzt.

Beispiele für die nucleophil substituierbare Abgangsgruppe X sind ortho-, meta- oder para-Toluolsulfonat, Methansulfonat, Trifluoracetat, Trifluormethansulfonat, Nonafluorbutylsulfonat, Benzolsulfonat, p-Nitrobenzolsulfonat, Cl, Br oder J.

Geeignete Basen sind beispielsweise NaOH, KOH, NaH, KH oder Trialkylamine. Bevorzugt sind NaH, KH,Triethylamin und KOH.

Die Umsetzung wird zweckmäßigerweise bei Temperaturen zwischen 20 und 100°C, bevorzugt zwischen 60 und 90°C durchgeführt. Da der Deprotonierungsschritt in der Regel exotherm verläuft, kann zu diesem Zeitpunkt der Synthese eine Kühlung zweckmäßig sein, beispielsweise auf 0 bis 20°C.

Das erfindungsgemäße Verfahren kann in Gegenwart oder Abwesenheit organischer Lösungsmittel durchgeführt werden. Als organische Lösungsmittel kommen insbesondere Dimethylformamid, Toluol oder Ethylacetat in Betracht. Es ist weiterhin vorteilhaft, die erfindungsgemäße Umsetzung unter Inertgasatmosphäre durchzuführen.

Die Herstellung von Verbindungen der Formel (II) kann nach literaturbekannten Methoden, beispielsweise gemäß O. Neunhoeffer et al., Chem. Ber. 94 (1961), 2514, erfolgen.

Die Verbindungen der Formel (I) eignen sich als Liganden in Metallkomplex-katalysierten organischen Reaktionen oder als Stabilisatoren für Polyalkylenglykole.

In den folgenden Beispielen bedeutet "DC" Dünnschichtchromatogramm, "GC" Gaschromatogramm, "EE" Ethylacetat und "DMF" Dimethylformamid.

### Beispiel 1

### Triphenylphosphin-4-yl-tri-ethylenglykolmethylether

| Ansatz: | | |
|---|---|---|
| 200 g (0,7195 mol) | p-Hydroxy-triphenylphosphan | 278 g/mol |
| 18 g (0,75 mol) | Natriumhydrid | 24 g/mol |
| 265 g (0.75 mol) | (p-Tosyl)-tri-ethylenglykolmethylether | (90 %ig) |

### Durchführung Schutzgastechnik

200 g p-Hydroxy-triphenylphosphan werden in 1 Liter DMF gelöst. Man versetzt langsam, portionsweise mit Natriumhydrid (exotherm), wobei Wasserstoffentwicklung einsetzt. Man rührt 1 Stunde bei Raumtemperatur.

Die Mischung wird mit 265 g (p-Tosyl)-tri-ethylenglykolmethylether (90 %ig) versetzt und 1 Stunde bei 80°C gerührt. Die Umsetzung wird im DC (Laufmittel: EE/n-Heptan = 1/1) kontrolliert.

### Aufarbeitung:

Das Lösungsmittel wird am Hochvakuum entfernt und der Rückstand mit 1 Liter Wasser und 2 Liter tert.-Butyl-methylether versetzt. Die organische Phase wird abgetrennt. Die wäßrige Phase wird zweimal mit je 500 ml tert.-Butyl-methylether ausgeschüttelt. Die vereinigten organischen Phasen werden über Na₂SO₄ getrocknet, filtriert und eingeengt.
- DC-Kontrolle:: Laufmittel EE/n-Heptan 1/1
Produkt R_{f} = 0,7
Tosyl-Ether: R_{f} = 0,4

Das Rohprodukt wird auf die 1,2-fache Gewichtsmenge Kieselgel aufgezogen und über eine Säule chromatographiert.
- Säulenchromatographie:: 2 kg Kieselgel, Laufmittel EE/n-Heptan 1/1
- Ausbeute:: ∼ 70 % d. Th. farbloses, sehr zähes Öl
- ³¹P-NMR (CD₂Cl₂):: -5,0 ppm

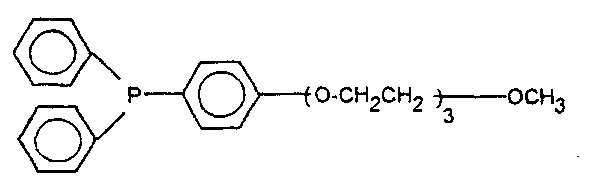

### Beispiel 2

### Triphenylphosphin-3-yl-tri-ethylenglykolmethylether

| Ansatz: | | |
|---|---|---|
| 200 g (0,7195 mol) | m-Hydroxy-triphenylphosphan | 278 g/mol |
| 18 g (0,75 mol) | Natriumhydrid | 24 g/mol |
| 265 g (0,75 mol) | (p-Tosyl)-tri-ethylenglykolmethylether | (90 %ig) |

### Durchführung: Schutzgastechnik

200 g m-Hydroxy-triphenylphosphan werden in 1 Liter DMF gelöst. Man versetzt langsam, portionsweise mit Natriumhydrid (exotherm), wobei Wasserstoffentwicklung einsetzt. Man rührt 1 Stunde bei Raumtemperatur.
Die Mischung wird mit 265 g (p-Tosyl)-tri-ethylenglykolmethylether (90 %ig) versetzt und 1 Stunde bei 80°C gerührt. Die Umsetzung wird im DC (Laufmittel: EE/n-Heptan = 1/1) kontrolliert.

### Aufarbeitung:

Das Lösungsmittel wird am Hochvakuum entfernt und der Rückstand mit 1 Liter Wasser und 2 Liter tert.-Butyl-methylether versetzt. Die organische Phase wird abgetrennt. Die wäßrige Phase wird zweimal mit je 500 ml tert.-Butyl-methylether ausgeschüttelt. Die vereinigten organischen Phasen werden über Na₂SO₄ getrocknet, filtriert und eingeengt.
- DC-Kontrolle:: Laufmittel EE/n-Heptan 1/1
Produkt: R_{f} = 0,7
Tosyl-Ether: R_{f} = 0,4

Das Rohprodukt wird auf die 1,2-fache Gewichtsmenge Kieselgel aufgezogen und über eine Kieselgelsäule chromatographiert.
- Säulenchromatographie:: 2 kg Kieselgel, Laufmittel EE/n-Heptan 1/1
- Ausbeute:: ∼ 70 % d. Th. farbloses, sehr zähes Öl
- ³¹P-NMR (CD₂Cl₂):: -5,1 ppm

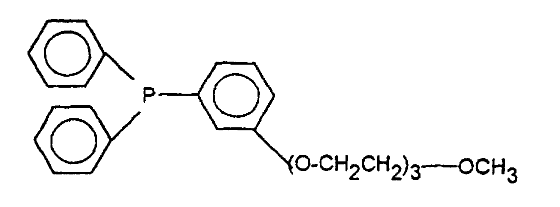

### Beispiel 3

### (Triphenylphosphin-2-y)l-tri-ethylenglykolmethylether

| Ansatz: | | |
|---|---|---|
| 200 g (0,7195 mol) | o-Hydroxy-triphenylphosphan | 278 g/mol |
| 18 g (0,75 mol) | Natriumhydrid | 24 g/mol |
| 265 g (0,75 mol) | (p-Tosyl)-tri-ethylenglykolmethylether | (90 %ig) |

### Durchführung: Schutzgastechnik

200 g o-Hydroxy-triphenylphosphan werden in 1 Liter DMF gelöst. Man versetzt langsam, portionsweise mit Natriumhydrid (exotherm), wobei Wasserstoffentwicklung einsetzt. Man rührt 1 Stunde bei Raumtemperatur.
Die Mischung wird mit 265 g (p-Tosyl)-tri-ethylenglykolmethylether (90 %ig) versetzt und 1 Stunde bei 80°C gerührt. Die Umsetzung wird im DC (Laufmittel: EE/n-Heptan = 1/1) kontrolliert.

### Aufarbeitung:

Das Lösungsmittel wird am Hochvakuum entfernt und der Rückstand mit 1 Liter Wasser und 2 Liter tert.-Butyl-methylether versetzt. Die organische Phase wird abgetrennt. Die wäßrige Phase wird zweimal mit je 500 ml tert.-Butyl-methylether ausgeschüttelt. Die vereinigten organischen Phasen werden über Na₂SO₄ getrocknet, filtriert und eingeengt.
- DC-Kontrolle:: Laufmittel EE/n-Heptan 1/1
Produkt: R_{f} = 0,7
Tosyl-Ether: R_{f} = 0,4

Das Rohprodukt wird auf die 1,2-fache Gewichtsmenge Kieselgel aufgezogen und über eine Kieselgelsäule chromatographiert:
- Säulenchromatographie:: 2 kg Kieselgel, Laufmittel EE/n-Heptan 1/1
- Ausbeute:: ∼ 70 % d. Th. farbloses, sehr zähes Öl
- ³¹P-NMR (CD₂Cl₂):: - 23 ppm

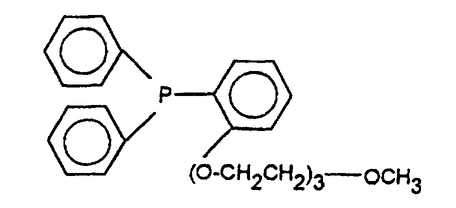

### Herstellung von p-Toluolsulfonyl-tri-ethylenglykol-methylether

200 g p-Toluolsulfonsäurechlorid und 172,4 g Triethylenglykolmonomethylether werden in 1 Liter Dichlormethan gelöst und auf 0°C gekühlt. Unter heftigem Rühren werden 236,3 g frisch gepulvertes Kaliumhydroxid so zudosiert, daß die Temperatur nicht über 5°C steigt. Die Mischung wird 3 Stunden bei 0°C gehalten. Man versetzt mit 1 Liter Dichlormethan und 1,2 Liter Eiswasser. Sollte weiterer Feststoff entstehen, werden weitere 0,5 Liter Eiswasser zugegeben.
Die organische Phase wird abgetrennt und mit 0,3 Liter gesättigter Kochsalzlösung gewaschen. Es wird über Na₂SO₄ getrocknet und im Vakuum zur Trockene eingeengt.
- Ausbeute:: 313,4 g; GC: 92,6 %ig

### Beispiel 4

### Herstellung von p-(P41/300)-triphenylphosphan

Unter P41/300 versteht man ein mit Ethylenoxid und Propylenoxid im molaren Verhältnis von 4:1 verethertes Pentaerythrit, wobei das Produkt eine Viskosität von 300 mPa·s hat und etwa 84 Ethoxy- und etwa 21 Propoxy-Gruppen aufweist.

28,33 g (101,8 mmol) p-Hydroxy-triphenylphosphan werden in 40 ml DMF gelöst. Man versetzt mit 101,8 mmol Kaliumhydroxid und erhitzt 1 Stunde auf 80°C. Anschließend versetzt man mit 509 g (102 mmol) P41/300-Tosylat und rührt 2 Stunden bei 80°C, wobei das DMF anschließend im Vakuum abgedampft wird.
Das Produkt ist vollständig löslich in Wasser bei 50°C. Es emulgiert in Wasser bei Temperaturen <20°C und >80°C. Der Prozeß ist reversibel.
- ³¹P-NMR (CD₂Cl₂):: - 5,2 ppm

### Beispiel 5

### Herstellung von m-(P41/300)-triphenylphosphan

28,33 g m-Hydroxy-triphenylphosphan werden in 40 ml DMF gelöst. Man versetzt mit 101,8 mmol Kaliumhydroxid und erhitzt 1 Stunde auf 80°C. Anschließend versetzt man mit 509 g (102 mmol) P41/300-TOS und rührt 2 Stunden bei 80°C. Das Produkt ist vollständig löslich in Wasser bei 50°C. Es emulgiert in Wasser bei Temperaturen <20°C und >80°C. Der Prozeß ist reversibel.
³¹P-NMR(CD₂Cl₂): -5,1 ppm

### Beispiel 6

### Herstellung von o-(P41/300)-triphenylphosphan

28,33 g o-Hydroxy-triphenylphosphan werden in 40 ml DMF gelöst. Man versetzt mit 101,8 mmol Kaliumhydroxid und erhitzt 1 Stunde auf 80°C. Anschließend versetzt man mit 509 g (102 mmol) P41/300-TOS und rührt 2 Stunden bei 80°C.
Das Produkt ist vollständig löslich in Wasser bei 50°C. Es emulgiert in Wasser bei Temperaturen <20°C und >80°C. Der Prozeß ist reversibel.
³¹P-NMR (CD₂Cl₂): - 23,5 ppm

### Herstellung von P41/300-Tosylat:

500 g P41/300 und 19,07 g p-Toluolsulfonylchlorid werden in 1 Liter Dichlormethan gelöst. Anschließend versetzt man mit 15,18 g Triethylamin. Es wird 2 Stunden unter Rückfluß erhitzt. Das Reaktionsgemisch wird mit 250 ml Wasser versetzt, die wäßrige von der organischen Phase getrennt und die organische Phase mit Na₂SO₄ getrocknet.
Ausbeute: 509,7 g

### Beispiele 7 bis 16

### Allgemeine Herstellungsvorschrift für die in den Beispielen 7 bis 16 verwendeten Einsatzstoffe

Nachfolgend seien die in der allgemeinen Vorschrift verwendeten Polyalkylenglykole näher erläutert.

Es ist unter PEG 200 ein Gemisch von Polyethylenglykolen der Formel H(OCH₂CH₂)ₘOH, worin m für eine ganze Zahl von 3 bis 6 steht, unter PEG 400 ein Gemisch von Polyethylenglykolen der Formel H(OCH₂CH₂)ₘOH, worin m für eine ganze Zahl von 7 bis 10 steht, unter PEG 600 ein Gemisch von Polyethylenglykolen der Formel H(OCH₂CH₂)ₘOH, worin m für eine ganze Zahl von 11 bis 16 steht, unter PEG 1000 ein Gemisch von Polyethylenglykolen der Formel H(OCH₂CH₂)ₘOH, worin m für eine ganze Zahl von 15 bis 30 steht, unter PEG 1500 ein Gemisch von Polyethylenglykolen der Formel H(OCH₂CH₂)ₘOH, worin m für eine ganze Zahl von 25 bis 35 steht, zu verstehen. Diesen Gemischen ist jeweils ein entsprechendes mittleres Molgewicht von 200 (PEG 200), etwa 400 (PEG 400), etwa 600 (PEG 600), 1000 (PEG 1000) respektive etwa 1500 (PEG 1500) zuzuordnen.

Es ist unter M 350 ein Gemisch von Verbindungen der Formel CH₃(OCH₂CH₂)ₘOH, worin m für eine ganze Zahl von 5 bis 9 steht, unter M 500 ein Gemisch von Verbindungen der Formel CH₃(OCH₂CH₂)ₘOH, worin m für eine ganze Zahl von 9 bis 13 steht, unter M 750 ein Gemisch von Verbindungen der Formel CH₃(OCH₂CH₂)ₘOH, worin m für eine ganze Zahl von 12 bis 20 steht, zu verstehen. Diesen Gemischen ist jeweils ein entsprechendes mittleres Molgewicht von etwa 350 (M 350), etwa 500 (M 500) respektive 750 (M 750) zuzuordnen.

Es ist unter M41/40 ein Gemisch von Verbindungen der Formel CH₃(OW)ₘOH zu verstehen, wobei m für eine ganze Zahl zwischen 18 und 25 steht und W für eine Gruppe der Formeln - CH₂CH₂ -, - CH(CH₃)CH₂ - oder - CH₂CH(CH₃) - steht, wobei das Verhältnis von Ethylen-gruppen (- CH₂CH₂ -) und Methylethylengruppen (- CH(CH₃)CH₂ - oder - CH₂CH(CH₃) -) 4:1 beträgt. Diesem Gemisch ist ein entsprechendes mittleres Molgewicht von 1000 zuzuordnen.

Es ist unter P41/300 ein Gemisch von Verbindungen der Formel C{(OW)ₘOH}₄ zu verstehen, wobei m für eine ganze Zahl zwischen 90 und 120 steht und W für eine Gruppe der Formeln - CH₂CH₂ -, - CH(CH₃)CH₂ - oder - CH₂CH(CH₃) - steht, wobei das Verhältnis von Ethylen-gruppen (- CH₂CH₂ -) und Methylethylengruppen (- CH(CH₃)CH₂ - oder - CH₂CH(CH₃) -) 4:1 beträgt. Diesem Gemisch ist ein entsprechendes mittleres Molgewicht von 5000 zuzuordnen.

Es ist unter B11/50 ein Gemisch von Verbindungen der Formel n-C₄H₉-(OW)ₘOH zu verstehen, wobei m für eine ganze Zahl zwischen 11 und 21 steht und W für eine Gruppe der Formeln - CH₂CH₂ -, - CH(CH₃)CH₂ - oder - CH₂CH(CH₃) - steht, wobei das Verhältnis von Ethylengruppen (- CH₂CH₂ -) und Methylethylengruppen (- CH(CH₃)CH₂ - oder - CH₂CH(CH₃) -) 1:1 beträgt. Diesem Gemisch ist ein entsprechendes mittleres Molgewicht von 1700 zuzuordnen.

In den Formelschemata ist für m jeweils ein Mittelwert angegeben, so daß die angegebene Formel das mittlere Molgewicht der jeweiligen Substanzklasse hat.

### 1. O-Mesyl-polyalkylenglykolether

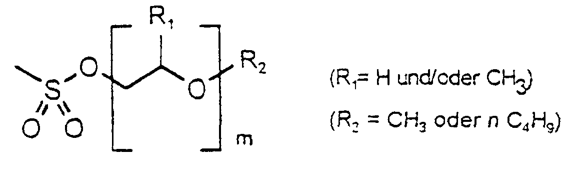

Allgemeine Vorschrift: 50 mMol Polyalkylenglykol, gelöst in 500 ml Dichlormethan, werden mit 4,7 g (60 mMol) Methansulfonsäurechlorid versetzt; anschließend tropft man bei Raumtemperatur 10,4 ml (75 mMol) Triethylamin unter Rühren zu. Unter Polyalkylenglykol seien die vorstehend erläuterten Verbindungen verstanden Die Reaktionslösung läßt man etwa 16 Stunden bei Raumtemperatur stehen [bei (e) 8,5 Stunden Rückfluß].

Zur Aufarbeitung und Reinigung wird die Reaktionslösung 3x mit je 100 ml 5 %iger wäßriger Citronensäure, 2x mit je 100 ml gesättigter Natriumhydrogencarbonatlösung und einmal mit 100 ml Wasser gewaschen. Anschließend trocknet man die Dichlormethanphase mit Magnesiumsulfat und dampft das Lösungsmittel ab. Zur Entfernung von Wasserspuren wird der Rückstand in Toluol aufgenommen, 2 Stunden am Rückfluß gekocht und Wasser mittels eines Wasserabscheiders ausgekreist. Nach dem Abdestillieren des Toluols kann das O-Mesyl-polyalkylenglykol weiter umgesetzt werden.
Zur Absicherung der Strukturen der Zielverbindungen und der Reinheiten wurden die Protonenverhältnisse in den ¹H-NMR-Spektren und die quantitative Bestimmung der OH-Konzentration durch die Acetanhydrid/Pyridin-Methode (Titration der dabei freiwerdenden Essigsäuremenge), dünnschichtchromatographische und HPLC-Methoden herangezogen.
(a) O-Mesyl-[M 350] 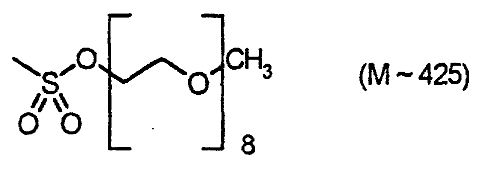 Einsatz: 0,2 Mol M 350, (Molgewicht » 350)
   Ausbeute: 83 g (97,5 %), dickflüssiges Öl
   DC, R_{F} = 0,6 (CH₂Cl₂:C₂H₅OH = 9:1)
   ¹H-NMR: (CDCl₃) δ [ppm] 3,08 (OSO₂CH₃, s 3H), 3,38 (OCH₃, s 3H), 3,52-4,40 (OCH₂, m ca.32)
   Keine freie OH-Funktion mehr vorhanden (Acetanhydrid/Pyridin-Methode)
(b) O-Mesyl-[M 500] 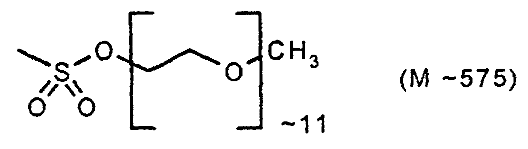 Einsatz: 0,2 Mol M 500, (Molgewicht » 500)
   Ausbeute: 111,6 g (97 %), dickflüssiges Öl
   DC, R_{F} = 0,5 (CH₂Cl₂: C₂H₅OH = 9:1)
   ¹H-NMR: (CDCl₃) δ [ppm] 3,08 (OSO₂CH₃, s 3H), 3,38 (OCH₃, s 3H), 3,52-4,40 (OCH₂, m ca.45H)
   Keine freie OH-Funktion mehr vorhanden (Acetanhydrid/Pyridin-Methode)
(c) O-Mesyl-[M 7501 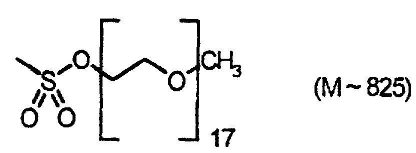 Einsatz: 0,1 Mol M 750, (Molgewicht » 750)
   Ausbeute: 82,8 g (97,5 %), dickflüssiges Öl
   DC, R_{F} = 0,59 (CH₂Cl₂:C₂H₅OH = 9:2)
   ¹H-NMR: (CDCl₃) δ [ppm] 3,08 (OSO₂CH₃, s 3H), 3,38 (OCH₃, s 3H), 3,52-4,40 (OCH₃, m ca.68H)
   Keine freie OH-Funktion mehr vorhanden (Acetanhydrid/Pyridin-Methode)
(d) O-Mesyl-[M 41/40] 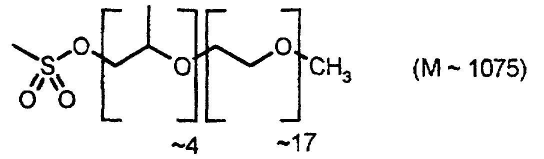 Einsatz: 0,1 Mol M41/40, (Molgewicht » 1000)
   Ausbeute: 99,6 g (92.6 %), dickflüssiges Öl
   ¹H-NMR: (CDCl₃) δ [ppm] 3,08 (OSO₂CH₃, s 3H), 3,38 (OCH₃, s 3H), 1,15 und 1,38 (CCH₃, 2d 12H), 3,35-4,40 (OCH₂ und OCH, m ca.80H)
   Keine freie OH-Funktion mehr vorhanden (Acetanhydrid/Pyridin-Methode)
(e) O-Mesyl-[B 11/50] 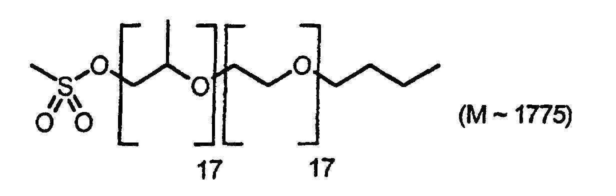 Einsatz: 0,1 Mol B 11/50, (Molgewicht » 1700)
   Ausbeute: 173 g (97 %), dickflüssiges Öl
   ¹H-NMR: (CDCl₃) δ [ppm] 0,92 (Bu CH₃, t 3H), 1,15 und 1,38 (CCH₃, CCH₂CH₂C, m 55H), 3,08 (OSO₂CH₃, "s" 3H), 3,35- 4,40 (OCH₂ und OCH, m ca. 121H)
   Keine freie OH-Funktion mehr vorhanden (Acetanhydrid/Pyridin-Methode)

### 2. O-Mesyl-polyethylenglykole

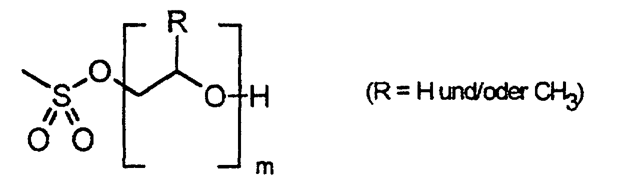

Allgemeine Vorschrift: 100 mMol Polyethylenglykol, gelöst in 1000 ml Dichlormethan, werden mit 11,46 g (100 mMol) Methansulfonsäurechlorid versetzt; anschließend tropft man bei 0°C 12,2 g (120 mMol) Triethylamin unter Rühren zu. Die Reaktionslösung läßt man etwa 16 Stunden bei Raumtemperatur stehen.
Zur Aufarbeitung wird die Reaktionslösung 3x mit je 200 ml 5 %iger wäßriger Citronensäure, 2x mit je 200 ml gesättigter Natriumhydrogencarbonatlösung und einmal mit 200 ml Wasser gewaschen. Anschließend trocknet man die Dichlormethanphase mit Magnesiumsulfat und dampft das Lösungsmittel ab. Das Reaktionsprodukt besteht aus einer Mischung von Ausgangspolyalkylenglykol, einfach- und zweifach-mesyliertem Polyalkylenglykol; das erwünschte einfachmesylierte Produkt kann durch Säulenchromatographie (Kieselgel) abgetrennt werden. (siehe Beispiele)

Zur Absicherung der Strukturen der Zielverbindungen und der Reinheiten wurden die Protonenverhältnisse in den ¹H-NMR-Spektren und die quantitative Bestimmung der OH-Konzentration durch die AcetanhydridlPyridin-Methode (Titration der dabei freiwerdenden Essigsäuremenge), dünnschichtchromatographische und HPLC-Methoden herangezogen.
Die Abkürzung R_{f} steht für den Retentionsquotienten in der Dünnschichtchromatographie (DC).
(a) O-Mesyl-[PEG 200] 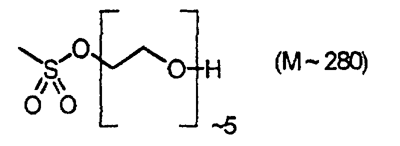 Einsatz: 0,5 Mol PEG 200, (Molgewicht » 200)
   Reinigung: SC [Kieselgel (φ=10 cm, h=100 cm)] CH₂Cl₂ dann CH₂Cl₂:C₂H₅OH=18:2
   Ausbeute: 24 g (17,3 %), dickflüssiges Öl
   DC, R_{F} = 0,37 (CH₂Cl₂:C₂H₅OH=9:1)
   ¹H-NMR: (CDCl₃) d [ppm] 2,70 (OH, s 1H), 3,08 (OSO₂CH₃, "s" 3H), 3,58-4,40 (OCH₂, m ca. 18H)
   Eine OH-Funktion/Molekül noch vorhanden (Acetanhydrid/Pyridin-Methode)
(b) O-Mesyl-[PEG 600] 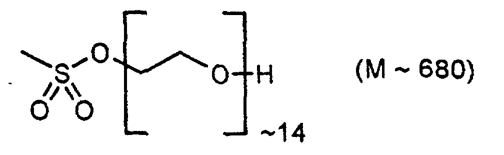 Einsatz: 0,17 Mol PEG 600, (Molgewicht » 600))
   Reinigung: SC [Kieselgel (φ=10 cm, h=100 cm)] CH₂Cl₂ dann CH₂Cl₂:C₂H₅OH=18:2
   Ausbeute: 29,5 g (26 %), dickflüssiges Öl
   DC, R_{F} = 0,36 (CH₂Cl₂:C₂H₅OH=9:1)
   ¹H-NMR: (CDCl₃) δ [ppm] 2,73 (OH, s 1H), 3,08 (OSO₂CH₃; s 3H), 3,59- 4,40 (OCH₂, m ca. 55H)
   Eine OH-Funktion/Molekül noch vorhanden (Acetanhydrid/Pyridin-Methode)
(c) O-Mesyl-[PEG 1000] 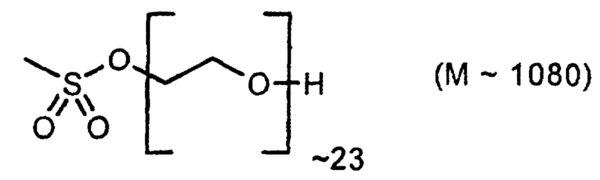 Einsatz: 0,1 Mol PEG 1000, (Molgewicht » 1000)
   Reinigung: SC [Kieselgel (φ=10 cm, h=100 cm)] Aceton
   Ausbeute: 32,2 g (30 %), wachsartiges Produkt
   DC, R_{F} = 0,34 (CH₂Cl₂:C₂H₅OH=5:1)
   ¹H-NMR: (CDCl₃) δ [ppm] 2,65 (OH, s 1H), 3,08 (OSO₂CH₃; s 3H), 3,57-4,40 (OCH₂, m ca. 92H)
   Eine OH-Funktion/Molekül noch vorhanden (Acetanhydrid/Pyridin-Methode)
(d) O-Mesyl-[PEG 15001 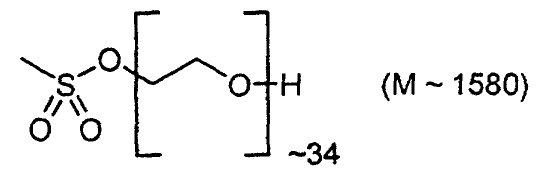 Einsatz: 0,1 Mol PEG 1500, (Molgewicht » 1500))
   Reinigung: SC [Kieselgel (φ=10 cm, h=100 cm)] Aceton
   Ausbeute 20 g, (19 %)
   DC, R_{F} = 0.45 (CH₂Cl₂:C₂H₅OH= 4:1)
   ¹H-NMR: (CDCl₃) δ [ppm] 2,65 (OH, s 1H), 3,08 (OSO₂CH₃; s 3H), 3,57-4,40 (OCH₂, m ca. 135H)
   Eine OH-Funktion/Molekül noch vorhanden (Acetanhydrid/Pyridin-Methode)
(e) O-Mesyl-[P 41/300] 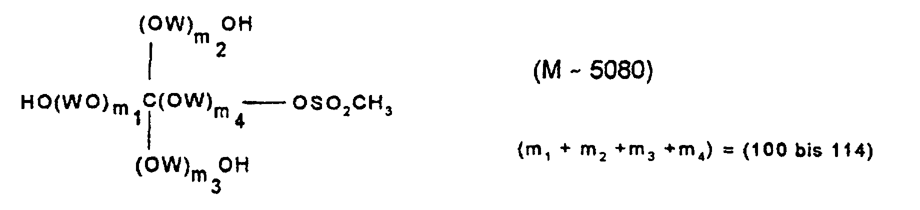 Einsatz: 0,01 Mol P 41/300, (Molgewicht » 5000)
   Reinigung: Wie in der Allgem.Vorschrift für O-Mesyl-polyalkylenglykole (1.)
   Ausbeute: 49,8 g (98 %), dickflüssiges Öl
   DC (RP 18), R_{F} = ca. 0,9 (CH₃OH:CH₃CN=7:3)
   ¹H-NMR: (CDCl₃) δ [ppm] 1,14 (CCH₃, m ca. 65H) 2,80 (OH, s 3H), 3,08 (OSO₂CH₃; s 3H), 3,20-4,40 (OCH₂, OCH, m ca. 420H)
   Das Reaktionsprodukt enthält statistisch verteilt noch 3 OH-Funktionen (Acetanhydrid/Pyridin-Methode)

### Allgemeine Vorschrift für die Synthese von 4-(Diphenylphosphin)-phenoxypolyalkylenglykolen und -ethern (Beispiele 7 bis 16)

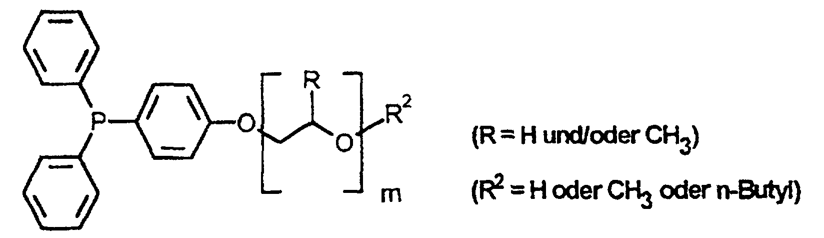

Allgemeine Vorschrift: Die folgende Reaktion wird unter Stickstoff ausgeführt. 1,73 g (60 mMol) NaH (80 %ig)werden in 150 ml DMF vorgelegt und 16,7 g (60 mMol) Diphenyl-4-hydroxyphenyl-phosphin gelöst in 100 ml DMF bei Raumtemperatur vorsichtig zugetropft. Wenn die Gasentwicklung beendet ist, setzt man 50 mMol O-Mesyl-polyalkylenglykol gelöst in 50 ml DMF zu und erwärmt 8 bis 10 Stunden auf 90-100°C.

Zur Aufarbeitung wird das DMF im Vakuum abgedampft, der Rückstand in 1000 ml Dichlormethan aufgenommen, 3x mit je 250 ml 0,2N Schwefelsäure und 2x mit je 250 ml Wasser gewaschen. Anschließend trocknet man die organische Phase über Magnesiumsulfat und dampft dann das Lösungsmittel ab.
Reinigung der Reaktionsprodukte: siehe Beispiele.

### Beispiel 7:

### Herstellung von 4-(Diphenylphosphin)-phenoxy-[M 350], Kurzbezeichnung M350-TPP

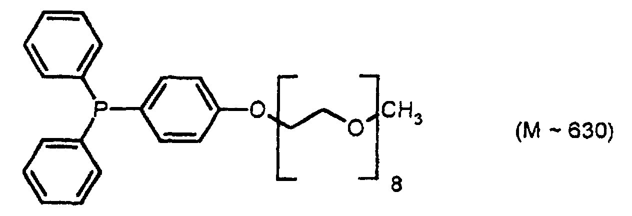

Einsatz: 0,05 Mol O-Mesyl-[M 350], (Molgewicht - 425)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=15:1
Ausbeute: 23,6 g (75 %), dickflüssiges Öl
DC, R_{F} = 0,3 (CH₂Cl₂:C₂H₅OH=15:1)
¹H-NMR; (CDCl₃) δ [ppm] 3,38 (OCH₃, s 3H), 3,52-4,20 (OCH₂, m ca. 32H), 6,80-7,80 (arom.H, m ca. 14H)
³¹P-NMR: 97% als Phosphin und 3 % als Phosphinoxid
S < 0,01 %

### Beispiel 8:

### Herstellung von 4-(Diphenylphosphin)-phenoxy-[M 500], Kurzbezeichnung (M 500-TPP)

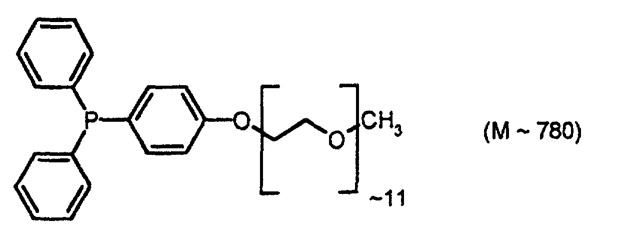

Einsatz: 0,05 Mol O-Mesyl-[M 500], (Molgewicht ∼ 575)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=15:1
Ausbeute: 32,3 g (83 %), dickflüssiges Öl
DC, R_{F} = 0,2 (CH₂Cl₂:C₂H₅OH=15:1)
¹H-NMR: (CDCl₃) δ [ppm] 3,38 (OCH₃, s 3H), 3,52- 4,20 (OCH₂, m ca. 45H), 6,80-7,80 (arom.H, m ca. 14H)
³¹P-NMR: 97% als Phosphin und 3 % als Phosphinoxid
S 0,02 %

### Beispiel 9:

### Herstellung von 4-(Diphenylphosphin)-phenoxy-[M 750], Kurzbezeichnung (M750-TPP)

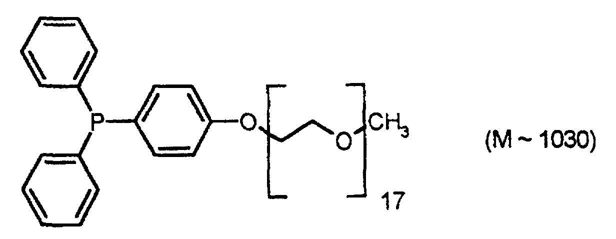

Einsatz: 0,04 Mol O-Mesyl-[M 750], (Molgewicht - 825)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=15:1
Ausbeute: 33,1 g (80 %), dickflüssiges Öl
DC, R_{F} = 0,27 (CH₂Cl₂:C₂H₅OH=15:1)
¹H-NMR: (CDCl₃) δ [ppm] 3,38 (OCH₃, s 3H), 3,52-4,20 (OCH₂, m ca. 69H), 6,80-7,80 (arom.H, m ca. 14H)
³¹P-NMR: 97% als Phosphin und 3 % als Phosphinoxid
S < 0,01 %

### Beispiel 10:

### Herstellung von 4-(Diphenylphosphin)-phenoxy-[M 41/40], Kurzbezeichnung (M41/40-TPP)

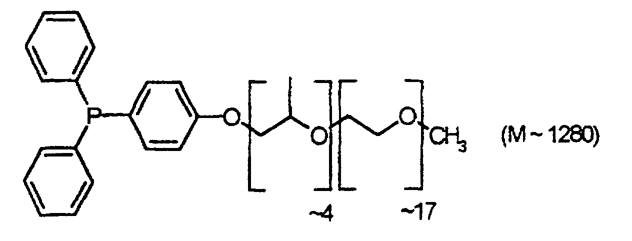

Einsatz: 0,0212 Mol O-Mesyl-[M 41/40], (Molgewicht - 1075)
Reinigung: SC [Kieselgel (φ 4,5 cm, h 30 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=9:1
Ausbeute: 18,6 g (68,5 %), dickflüssiges Öl
DC, R_{F} = 0,21 (CH₂Cl₂:C₂H₅OH=9:1)
¹H-NMR: (CDCl₃) δ [ppm] 1,15 und 1,30 (CCH₃, 2d 12H), 3,38 (OCH₃, s 3H), 3,36-4,20 (OCH₂, m ca. 80H), 6,80-7,80 (arom.H, m ca. 14H)
³¹P-NMR: 93% als Phosphin und 7 % als Phosphinoxid
S 0,08 %

### Beispiel 11:

### Herstellung von 4-(Diphenylphosphin)-phenoxy-[B 11/50], Kurzbezeichnung (B11/50-TPP)

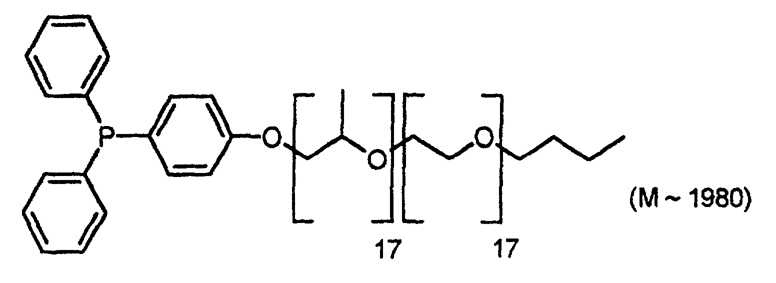

Einsatz: 0,02 Mol O-Mesyl-[B 11/50], (Molgewicht - 1775)
Reinigung: SC [Kieselgel (φ 4,5 cm, h 30 cm)] zunächst Ethylacetat zur Abtrennung von Diphenyl-4-hydroxyphenyl-phosphin und dessen Phosphinoxid, dann Ethylacetat:C₂H₅OH=20:1
Ausbeute: 13,8 g (35 %), dickflüssiges Öl
¹H-NMR: (CDCl₃) δ [ppm] 0,92 (Bu CH₃, t 3H), 1,15 und 1,38 (CCH₃, CCH₂CH₂C, m 55H), 3,35-4,40 (OCH₂ und OCH, m ca. 121H) 6,80-7,80 (arom.H, m ca. 14H)
³¹P-NMR: 82% als Phosphin und 18 % als Phosphinoxid
S < 0,01 %

### Beispiel 12:

### Herstellung von 4-(Diphenylphosphin)-phenoxy-[PEG 200], Kurzbezeichnung (PEG 200-TPP)

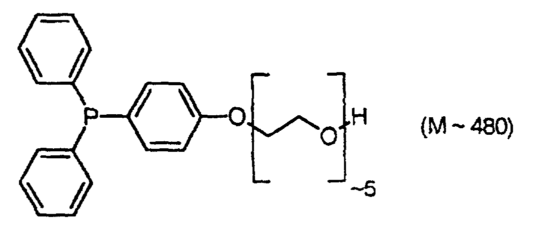

Einsatz: 0,054 Mol O-Mesyl-[PEG 200], (Molgewicht ∼ 280)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann Ethylacetat:C₂H₅OH=9:1
Ausbeute: 19,0 g (75 %), dickflüssiges Öl
DC, R_{F} = 0,45 (CH₂Cl₂:C₂H₅OH=9:1)
¹H-NMR: (CDCl₃) δ [ppm]) 2,80 (OH, br. s 1H), 3,52- 4,20 (OCH₂, m ca. 20H), 6,86-7,70 (arom.H, m ca. 14H)
³¹P-NMR: 96% als Phosphin und 4 % als Phosphinoxid
S < 0,01 %

### Beispiel 13:

### Herstellung von 4-(Diphenylphosphin)-phenoxy-[PEG 600], Kurzbezeichnung (PEG 600-TPP)

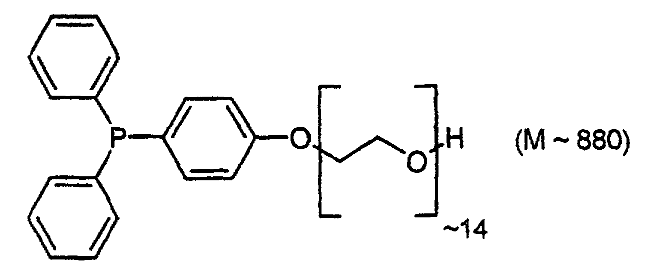

Einsatz: 0,022 Mol O-Mesyl-[PEG 600], (Molgewicht - 680)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=18:1
Ausbeute: 12,0 g (63 %), dickflüssiges Öl
DC, R_{F} = 0,35 (CH₂Cl₂:C₂H₅OH=9:1)
¹H-NMR: (CDCl₃) δ [ppm]) 2,80 (OH, br. s 1H), 3,56-4,20 (OCH₂, m ca. 55H), 6,86-7,70 (arom.H, m ca. 14H)
³¹P-NMR: 97% als Phosphin und 3 % als Phosphinoxid
S < 0,01 %

### Beispiel 14:

### Herstellung von 4-(Diphenylphosphin)-phenoxy-[PEG 1000], Kurzbezeichnung (PEG 1000-TPP)

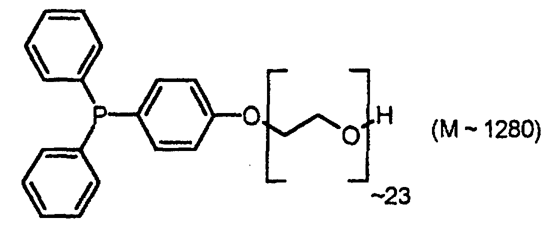

Einsatz: 0,015 Mol O-Mesyl-[PEG 1000], (Molgewicht ∼ 1080)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=10:2
Ausbeute: 9,2 g (50 %), dickflüssiges Öl
DC, R_{F} = .0,32 (CH₂Cl₂:C₂H₅OH=6:1)
¹H-NMR: (CDCl₃) δ [ppm]) 2,80 (OH, br. s 1H), 3,56-4,20 (OCH₂, m ca. 75H), 6,86-7,70 (arom.H, m ca. 14H) (Protonenzahl für PEG 1000-Teil zu niedrig, da bei der chromatographischen Reinigung bevorzugt die niedrigen Molekulargewichtsanteile isoliert wurden; durch HPLC-Analyse abgesichert, daß im Produkt keine 2 Phosphinreste pro Molekül enthalten sind)
³¹P-NMR: 96 % als Phosphin und 4 % als Phosphinoxid
S < 0,01 %

### Beispiel 15:

### Herstellung von 4-(Diphenylphosphin)-phenoxy-[PEG 1500], Kurzbezeichnung (PEG 1500-TPP)

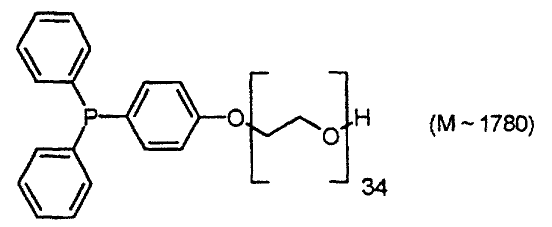

Einsatz: 0,0126 Mol O-Mesyl-[PEG 1500], (Molgewicht - 1580)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=9:1
Ausbeute: 10,2 g (46 %), wachsartiges Produkt
DC, R_{F} = 0,35 (CH₂Cl₂:C₂H₅OH=6:1)
¹H-NMR: (CDCl₃) δ [ppm]) 2,80 (OH, br, s 1H), 3,56-4,20 (OCH₂, m ca. 136H), 6,86-7,70 (arom.H, m ca. 14H)
³¹P-NMR: 93% als Phosphin und 7 % als Phosphinoxid
S < 0,01 %

### Beispiel 16:

### Herstellung von 4-(Diphenylphosphin)-phenoxy-[P 41/300], Kurzbezeichnung (P41/300-TPP)

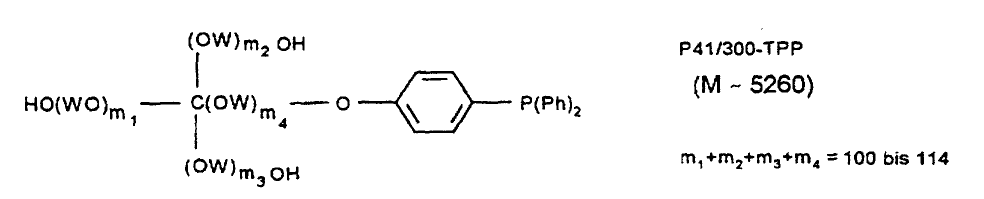

Einsatz: 0,01 Mol O-Mesyl-[P 41/300], (Molgewicht - 5080)
Reinigung: SC [Kieselgel (φ 4,5 cm, h 50 cm)] zunächst Ethylacetat zur Abtrennung von Diphenyl-4-hydroxyphenyl-phosphin und dessen Phosphinoxid, dann CH₂Cl₂ zur Entfernung von Ethylacetat, dann CH₂Cl₂:C₂H₅OH=10:1
Ausbeute: 10,2 g (46 %), dickflüssiges Öl
DC, R_{F} = 0,56 (CH₂Cl₂:C₂H₅OH=3:1)
¹H-NMR: (CDCl₃) δ [ppm]) 1,14 (CCH₃, m ca. 65H), 3,56-4,20 (OCH₂, OCH, m ca. 420H), 6,86-7,70 (arom.H, m ca. 14H)
³¹P-NMR: 82% als Phosphin und 18 % als Phosphinoxid
S < 0,01 %, P 0,52 %

## Patentansprüche

1. Verbindung der Formel (I) 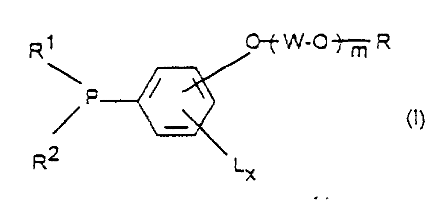 worin
m eine Zahl von 2 bis 300;
x eine Zahl von 0 bis 4;
W eine Gruppe der Formeln -CH₂-CH₂-, -CH(CH₃)CH₂- oder -CH₂CH(CH₃)-;
R Wasserstoff, ein geradkettiger oder verzweigter C₁-C₅-Alkylrest;
oder eine Gruppe der Formeln 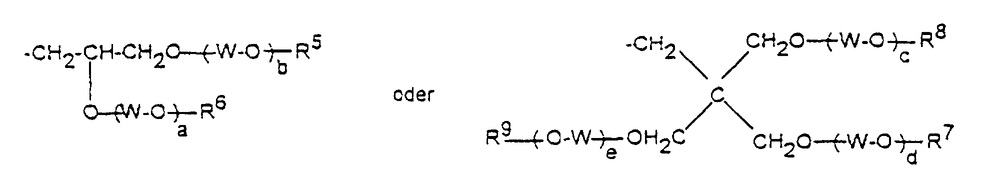 wobei
a, b, c, d und e unabhängig voneinander eine Zahl von 0 bis 1000, wobei mindestens eine der Zahlen a, b, c, d und e größer als 0 ist;
R⁵, R⁶, R⁷, R⁸ und R⁹ gleich oder verschieden sind und Wasserstoff, C₁-C₅-Alkyl oder eine Gruppe der Formel 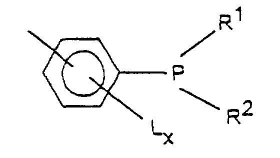 ist,
R¹ und R² gleich oder verschieden sind und einen geradkettigen, verzweigten oder cyclischen C₁-C₃₀-Alkylrest oder C₆-C₁₀-Arylrest, der unsubstituiert oder durch ein bis fünf C₁-C₃-Alkylreste substituiert ist, oder R¹ und R² zusammen mit dem dreiwertigen P-Atom ein Dibenzophospholyl der Formel 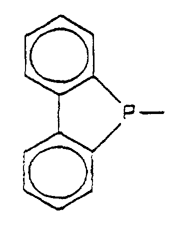 oder ein 3,4-Dimethylphospholyl der Formel 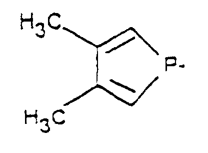 bilden, und
L C₁-C₅-Alkyl, C₁-C₅-Alkoxy, NO₂, NR³R⁴, wobei R³ und R⁴ unabhängig voneinander Wasserstoff oder C₁-C₄-Alkyl bedeuten, Cl oder OH bedeuten.

2. Verbindung nach Anspruch 1, **dadurch gekennzeichnet, daß** R Wasserstoff, Methyl, Ethyl, n-Propyl, n-Butyl oder eine Gruppe der Formel 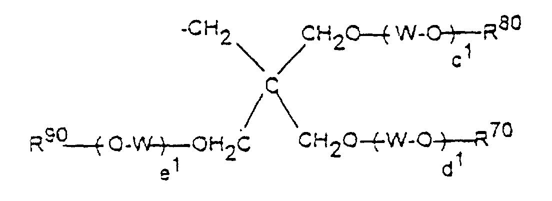 worin c¹, d¹ und e¹ unabhängig voneinander eine Zahl von 1 bis 500, insbesondere 2 bis 300, und
R⁷⁰, R⁸⁰ und R⁹⁰ gleich oder verschieden sind und Wasserstoff, Methyl, Ethyl, n-Propyl oder n-Butyl bedeuten.

3. Verbindung nach Anspruch 1 oder 2, **dadurch gekennzeichnet, daß** R¹ und R² gleich sind und jeweils einen geradkettigen oder verzweigten C₁-C₆-Alkylrest, einen Cyclohexylrest oder einen Phenylrest bedeuten.

4. Verbindung nach mindestens einem der Ansprüche 1 bis 3, **dadurch gekennzeichnet, daß** L Methoxy, Ethoxy Methyl, Ethyl oder OH bedeutet.

5. Verbindung nach mindestens einem der Ansprüche 1 bis 3, **dadurch gekennzeichnet, daß** x die Zahl 0 bedeutet.

6. Verbindung nach mindestens einem der Ansprüche 1 bis 5, **dadurch gekennzeichnet, daß** m eine Zahl von 2 bis 100 bedeutet.

7. Verfahren zur Herstellung einer Verbindung der Formel (I) gemäß einem oder mehreren der Ansprüche 1 bis 6, **dadurch gekennzeichnet, daß** man ein Hydroxyphenylphosphan der Formel (II) 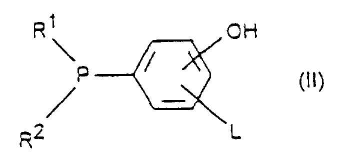 mit einer Base zum entsprechenden Phenolat deprotoniert und mit einer Verbindung der Formel (III)
X-(-W-O-)ₘ-R (III),
worin bedeutet:
X eine nucleophil substituierbare Abgangsgruppe;
m eine Zahl von 2 bis 300, bevorzugt 2 bis 100;
W eine Gruppe der Formeln -CH₂-CH₂-, -CH(CH₃)CH₂- oder -CH₂CH(CH₃)-;
R Wasserstoff, ein geradkettiger oder verzweigter C₁-C₅-Alkylrest;
oder eine Gruppe der Formeln 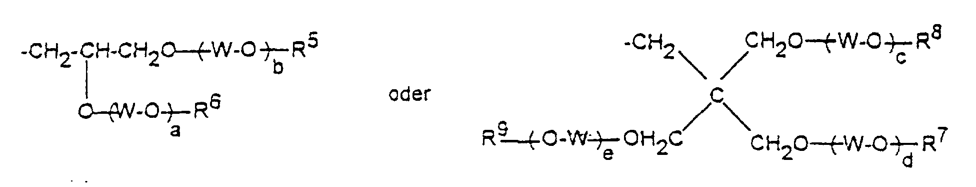 wobei
a, b, c, d und e unabhängig voneinander eine Zahl von 0 bis 1000, wobei mindestens eine der Zahlen a, b, c, d und e größer als 0 ist;
R⁵, R⁶, R⁷, R⁸ und R⁹ gleich oder verschieden sind und Wasserstoff, C₁-C₅-Alkyl oder eine Gruppe der Formel 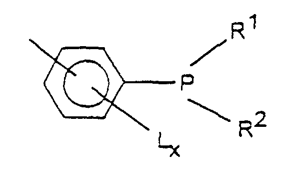 ist,
R¹ und R² gleich oder verschieden sind und einen geradkettigen, verzweigten oder cyclischen C₁-C₃₀-Alkylrest oder C₆-C₁₀-Arylrest, der unsubstituiert oder durch ein bis fünf C₁-C₃-Alkylreste substituiert ist, oder R¹ und R² zusammen mit dem dreiwertigen P-Atom ein Dibenzophospholyl der Formel 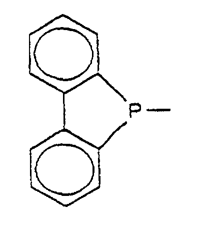 oder ein 3,4-Dimethylphospholyl der Formel 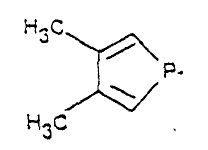 bilden, und
L C₁-C₅-Alkyl, C₁-C₅-Alkoxy NO₂, NR³R⁴, wcbei R³ und R⁴ unabhängig voneinander Wasserstoff oder C₁-C₄-Alkyl bedeuten, Cl oder OH bedeuten
zur Verbindung der Formel (I) umsetzt.

8. Verfahren nach Anspruch 7, **dadurch gekennzeichnet daß** X ein ortho-, meta- oder para-Toluolsulfonat, Methansulfonat, Trifluoracetat, Trifluormethansulfonat, Nonafluorbutylsulfonat, Benzolsulfonat, p-Nitrobenzolsulfonat, Cl, Br oder J ist.

9. Verfahren nach Anspruch 7 oder 8, **dadurch gekennzeichnet, daß** die Umsetzung in Gegenwart oder in Abwesenheit eines organischen Lösungsmittels durchgeführt wird.

10. Verwendung einer Verbindung nach mindestens einem der Ansprüche 1 bis 6 als Katalysatorbestandteil in Metallkomplex-katalysierten organischen Reaktionen.

## Claims

1. A compound of the formula (I) 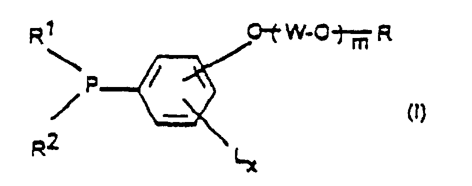 in which
m is a number from 2 to 300;
x is a number from 0 to 4;
W is a group of the formulae -CH₂-CH₂-, -CH(CH₃)CH₂- or -CH₂CH(CH₃)-;
R is hydrogen, a straight-chain or branched C₁-C₅-alkyl radical; or a group of the formulae 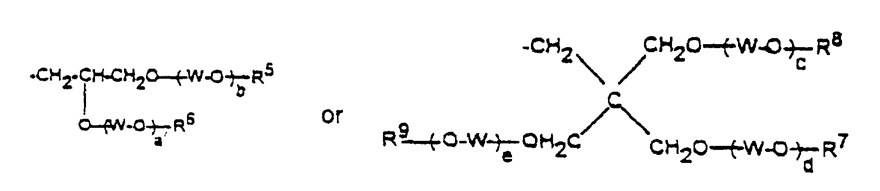 where
a, b, c, d and e independently of one another are numbers from 0 to 1000, at least one of the numbers a, b, c, d and e being greater than 0;
R⁵, R⁶, R⁷, R⁸ and R⁹ are identical or different and are hydrogen, C₁-C₅-alkyl or a group of the formula 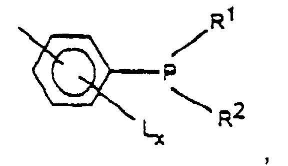
R¹ and R² are identical or different and are a straight-chain, branched or cyclic C₁-C₃₀-alkyl radical or C₆-C₁₀-aryl radical, which is unsubstituted or substituted by from one to five C₁-C₃-alkyl radicals, or R¹ and R², together with the trivalent P atom, form a dibenzophospholyl of the formula 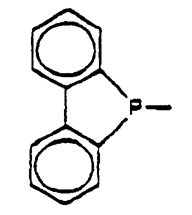 or a 3,4-dimethylphospholyl of the formula 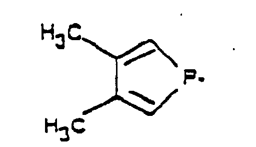 and
L is C₁-C₅-alkyl, C₁-C₅-alkoxy, NO₂, NR³R⁴, where R³ and R⁴ independently of one another are hydrogen or C₁-C₄-alkyl, or L is Cl or OH.

2. A compound as claimed in claim 1, wherein R is hydrogen, methyl, ethyl, n-propyl, n-butyl or a group of the formula 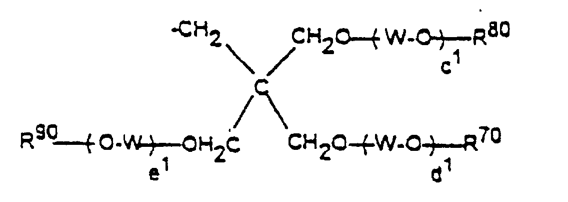 in which c¹, d¹ and e¹ independently of one another are a number from 1 to 500, in particular from 2 to 300, and
R⁷⁰, R⁸⁰ and R⁹⁰ are identical or different and are hydrogen, methyl, ethyl, n-propyl or n-butyl.

3. A compound as claimed in claim 1 or 2, wherein R¹ and R² are identical and are each a straight-chain or branched C₁-C₆-alkyl radical, a cyclohexyl radical or a phenyl radical.

4. A compound as claimed in at least one of claims 1 to 3, wherein L is methoxy, ethoxy, methyl, ethyl or OH.

5. A compound as claimed in at least one of claims 1 to 3, wherein x is 0.

6. A compound as claimed in at least one of claims 1 to 5, wherein m is a number from 2 to 100.

7. A process for the preparation of a compound of the formula (I) as claimed in one or more of claims 1 to 6, which comprises deprotonating a hydroxyphenylphosphine of the formula (II) 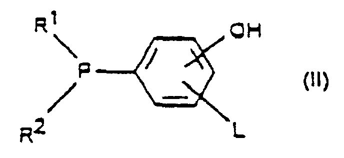 using a base to give the corresponding phenoxide, and reacting it with a compound of the formula (III)
X-(-W-O-)ₘ-R (III),
in which
X is a nucleophilically substitutable leaving group
m is a number from 2 to 300, preferably from 2 to 100;
W is a group of the formulae -CH₂-CH₂-, -CH(CH₃)CH₂- or -CH₂CH(CH₃)-;
R is hydrogen, a straight-chain or branched C₁-C₅-alkyl radical; or a group of the formulae 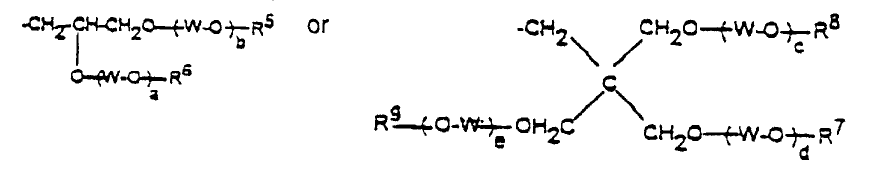 where
a, b, c, d and e independently of one another are numbers from 0 to 1000, at least one of the numbers a, b, c, d and e being greater than 0;
R⁵, R⁶, R⁷, R⁸ and R⁹ are identical or different and are hydrogen, C₁-C₅-alkyl or a group of the formula 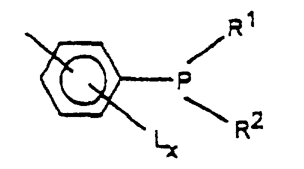
R¹ and R² are identical or different and are a straight-chain, branched or cyclic C₁-C₃₀-alkyl radical or C₆-C₁₀-aryl radical, which is unsubstituted or substituted by from one to five C₁-C₃-alkyl radicals, or R¹ and R², together with the trivalent P atom, form a dibenzophospholyl of the formula 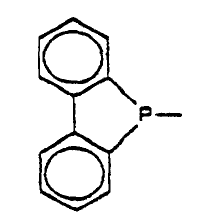 or a 3,4-dimethylphospholyl of the formula 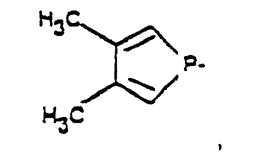 and
L is C₁-C₅-alkyl, C₁-C₅-alkoxy, NO₂, NR³R⁴, where R³ and R⁴ independently of one another are hydrogen or C₁-C₄-alkyl, or L is Cl or OH,
to give the compound of the formula (I).

8. The process as claimed in claim 7, wherein X is an ortho-, meta- or para-toluenesulfonate, methanesulfonate, trifluoroacetate, trifluoromethanesulfonate, nonafluorobutylsulfonate, benzenesulfonate, p-nitrobenzenesulfonate, Cl, Br or I.

9. The process as claimed in claim 7 or 8, wherein the reaction is carried out in the presence or in the absence of an organic solvent.

10. The use of a compound as claimed in at least one of claims 1 to 6 as catalyst constituent in metal-complex-catalyzed organic reactions.

## Revendications

1. Composé de formule (I) 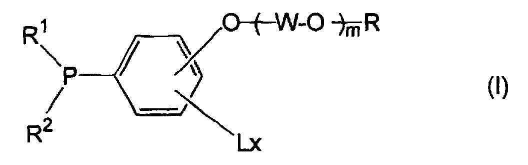 dans laquelle
m est un nombre de 2 à 300 ;
x est un nombre de 0 à 4 ;
W est un groupe de formule -CH₂-CH₂-, -CH(CH₃)CH₂- ou -CH₂CH(CH₃)- ;
R est l'hydrogène, un radical alkyle en C₁-C₅ linéaire ou ramifié ;
ou un groupe de formule 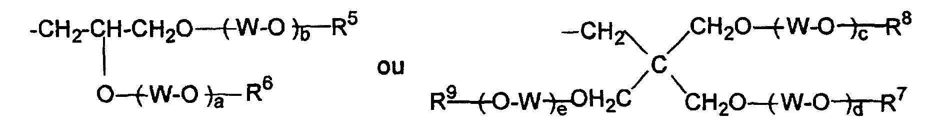 où
a, b, c, d et e indépendamment l'un de l'autre sont un nombre de 0 à 1000, au moins un des nombres a, b, c, d et e étant supérieur à 0 ;
R⁵, R⁶, R⁷, R⁸ et R⁹ sont semblables ou différents et sont l'hydrogène, un radical alkyle en C₁-C₅ ou un groupe de formule 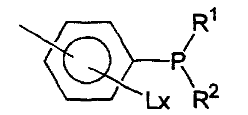
R¹ et R² sont semblables ou différents et sont un radical alkyle en C₁-C₃₀ linéaire, ramifié ou cyclique ou un radical aryle en C₆-C₁₀, qui n'est pas substitué ou qui est substitué par un à cinq radicaux alkyle en C₁-C₃, ou R¹ et R² forment avec l'atome de P trivalent un radical dibenzophospholyle de formule 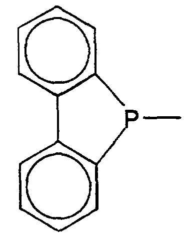 ou un radical 3,4-diméthylphospholyle de formule 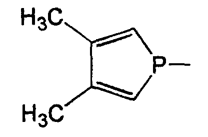
et
L est un radical alkyle en C₁-C₅, alcoxy en C₁-C₅, NO₂, NR³R⁴, où R³ et R⁴ indépendamment l'un de l'autre sont l'hydrogène ou un radical alkyle en C₁-C₄, Cl ou OH.

2. Composé selon la revendication 1, **caractérisé en ce que** R est l'hydrogène, un groupe méthyle, éthyle, n-propyle, n-butyle ou un groupe de formule 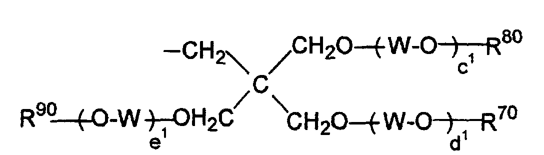 dans laquelle c¹, d¹ et e¹ indépendamment l'un de l'autre sont un nombre de 1 à 500, en particulier 2 à 300, et
R⁷⁰, R⁸⁰ et R⁹⁰ sont identiques ou différents et sont l'hydrogène, un groupe méthyle, éthyle, n-propyle ou n-butyle.

3. Composé selon la revendication 1 ou 2,**caractérisé en ce que** R¹ et R² sont identiques et chaque fois un radical alkyle en C₁-C₆ à chaîne linéaire ou ramifiée, un radical cyclohexyle ou un radical phényle.

4. Composé selon au moins une des revendications 1 à 3, **caractérisé en ce que** L est un radical méthoxy, éthoxy, méthyle, éthyle ou OH.

5. Composé selon au moins une des revendications 1 à 3, **caractérisé en ce que** x est le nombre 0.

6. Composé selon au moins une des revendications 1 à 5, **caractérisé en ce que** m est un nombre de 2 à 100.

7. Procédé de préparation d'un composé de formule (I) qui comprend la déprotonation d'une hydroxyphénylphosphine de formule (II) 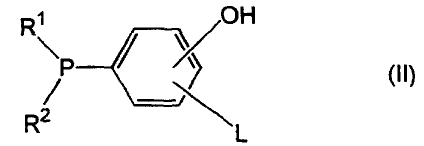 en utilisant une base pour donner le phénate correspondant, et la réaction de celui-ci avec un composé de formule (III)
X-(-W-O-)ₘ-R (III)
dans laquelle
X est un groupe partant pouvant être substitué de manière nucléophile;
m est un nombre de 2 à 300, de préférence 2 à 100 ;
W est un groupe de formule -CH₂-CH₂-, -CH(CH₃)CH₂- ou -CH₂CH(CH₃)- ;
R est l'hydrogène, un radical alkyle en C₁-C₅ linéaire ou ramifié ; ou un groupe de formule 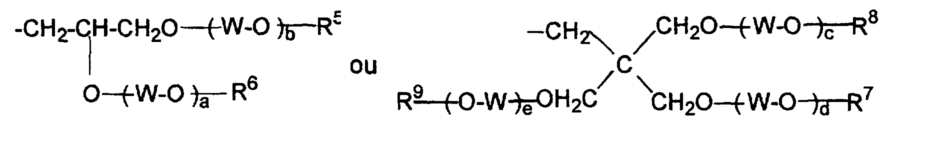 où
a, b, c, d et e indépendamment l'un de l'autre sont un nombre de 0 à 1000, au moins un des nombres a, b, c, d et e étant supérieur à 0 ;
R⁵, R⁶, R⁷, R⁸ et R⁹ sont semblables ou différents et sont l'hydrogène, un radical alkyle en C₁-C₅ ou un groupe de formule 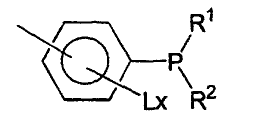
R¹ et R² sont semblables ou différents et sont un radical alkyle en C₁-C₃₀ linéaire, ramifié ou cyclique ou un radical aryle en C₆-C₁₀, qui n'est pas substitué ou qui est substitué par un à cinq radicaux alkyle en C₁-C₃, ou R₁ et R₂ forment avec l'atome de P trivalent un radical dibenzophospholyle de formule 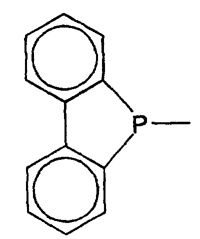 ou un radical 3,4-diméthylphospholyle de formule 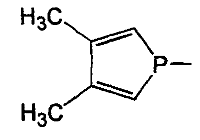
et
L est un radical alkyle en C₁-C₅, alcoxy en C₁-C₅, NO₂, NR³R⁴, où R³ et R⁴ indépendamment l'un de l'autre sont l'hydrogène ou un radical alkyle en C₁-C₄, Cl ou OH, pour obtenir le composé de formule (I).

8. Procédé selon la revendication 7, **caractérisé en ce que** X est l'ortho-, le méta- ou le para-toluènesulfonate, le méthanesulfonate, le trifluoroacétate, le trifluorométhanesulfonate, le nonafluorobutylsulfonate, le benzènesulfonate, le p-nitrobenzènesulfonate, Cl, Br ou I.

9. Procédé selon la revendication 7 ou 8, **caractérisé en ce qu'**on effectue la réaction en présence ou en l'absence d'un solvant organique.

10. Utilisation d'un composé selon au moins une des revendications 1 à 6 comme composant de catalyseur dans des réactions organiques catalysées par des complexes métalliques.
